# EUROPEAN PATENT SPECIFICATION

(11) **EP 1 933 921 B1**
(45) Date of publication and mention of the grant of the patent: **13.07.2016**
(21) Application number: 06836261.5
(22) Date of filing: 11.10.2006
(51) Int. Cl.: A61M 25/00, A61M 25/09

(54) **MEDICAL DEVICE COIL**
SPIRALE FÜR MEDIZINISCHE VORRICHTUNG
ENROULEMENT POUR DISPOSITIF MÉDICAL

(30) Priority: 11.10.2005 US 250079
(43) Date of publication of application: 25.06.2008
(73) Proprietor: Boston Scientific Limited, Hamilton HM11 (BM)
(72) Inventor: SHARROW, James S., Bloomington, MN 55431 (US)
(74) Representative: Vossius & Partner Patentanwälte Rechtsanwälte mbB
(86) International application number: PCT/US2006/039675
(87) International publication number: WO 2007/044783

(56) References cited:
- WO-A-98/56448
- DE-A1- 2 150 595
- US-A- 5 746 696
- US-A- 5 951 539
- US-A1- 2004 122 340

## Description

### Technical Field

The invention pertains generally to medical device coils useful for a variety of applications such as in guidewires, catheters, and the like. See for example US 5746696.

### Background

A wide variety of medical devices such as catheters and guidewires have been developed. Medical devices such as guidewires can be used in conjunction with devices such as catheters to facilitate navigation through the anatomy of a patient. Because the anatomy of a patient may be very tortuous, it can be desirable to have particular performance features in an elongate medical device. A number of different structures and assemblies for elongate medical devices such as guidewires are known each having certain advantages and disadvantages. However, there is an ongoing need to provide alternative structures and assemblies.

### Summary of Some Embodiments

The invention provides several alternative designs, materials and methods of manufacturing alternative medical device structures and assemblies.

Accordingly, an example embodiment of the invention can be found in an intracorporeal device including an elongate shaft, a first coil having a first coil length and a first coil lumen disposed about at least a portion of the elongate shaft, the first coil wrapped in a first direction, and a second coil disposed within the first coil lumen. The second coil is wrapped in a second direction opposite the first direction. The first coil is affixed to the second coil with a plurality of affixation points along the first coil length. In some embodiments, one or more additional coils may be so disposed and attached within the first coil lumen.

Another example embodiment of the invention can be found in a guidewire including a core wire having a tapered distal region, a first coil having a first coil length and a first coil lumen disposed about at least a portion of the tapered distal region, the first coil wrapped in a first direction, and a second coil having a second coil lumen disposed within the first coil lumen, the second coil wrapped in a second direction opposite the first direction. The first coil is affixed to the second coil with a plurality of affixation points along the first coil length. In some embodiments, one or more additional coils may be so disposed and attached within the first coil lumen.

Another example embodiment of the invention can be found in a method of manufacturing an intracorporeal device including forming one or more inner coils around a portion of an elongate shaft, the one or more inner coils including at least one inner coil wrapped in a first direction and having an inner coil length. Forming an outer coil around the one or more inner coils, the outer coil wrapped in a second direction opposite the first direction and affixing the one or more inner coils to the outer coil with a plurality of affixation points along the inner coil length.

The above summary of some embodiments is not intended to describe each disclosed embodiment or every implementation of the present invention. The Figures, and Detailed Description which follow more particularly exemplify these embodiments.

### Brief Description of the Figures

The invention may be more completely understood in consideration of the following detailed description of various embodiments of the invention in connection with the accompanying drawings, in which:
Figure 1 is a perspective view of an example coil incorporated into an elongate intracorporeal device;
Figure 2 is a side elevation view of an example coil;
Figure 3 is a cross-sectional view of an example coil shown in Figure 2;
Figure 4 is a side elevation view of an example coil;
Figure 5 is a cross-sectional view of an example coil shown in Figure 4;
Figure 6 is a side elevation view of an example coil;
Figure 7 is a cross-sectional view of an example coil shown in Figure 6;
Figure 8 is a side elevation view of an example coil;
Figure 9 is a cross-sectional view of an example coil shown in Figure 8;
Figure 10 is a cross-sectional view of an alternative example of a guidewire with an example coil;
Figure 11 is a cross-sectional view of an alternative guidewire with a example coil;
Figure 12 is a side elevation view of an example coil; and
Figure 13 is a cross-sectional view of an example coil shown in Figure 12.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description of Some Embodiments

For the following defined terms, these definitions shall be applied, unless a different definition is given in the claims or elsewhere in this specification.

The term "polymer" will be understood to include polymers, copolymers (e.g., polymers formed using two or more different monomers), oligomers and combinations thereof, as well as polymers, oligomers, or copolymers that can be formed in a miscible blend by, for example, coextrusion or reaction, including transesterification. Both block and random copolymers are included, unless indicated otherwise.

All numeric values are herein assumed to be modified by the term "about", whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The drawings, which are not necessarily to scale, depict illustrative embodiments of the claimed invention. For example, although discussed with specific reference to guidewires in the particular embodiments described herein, the invention may be applicable to a variety of medical devices that are adapted to be advanced into the anatomy of a patient through an opening or lumen. For example, the invention may be applicable to fixed wire devices, catheters (e.g. guide, balloon, stent delivery, etc.), drive shafts for rotational devices such as atherectomy catheters and IVUS catheters, endoscopic devices, laproscopic devices, embolic protection devices, spinal or cranial navigational devices, and other such devices. Additionally, while some embodiments may be adapted or configured for use within the vasculature of a patient, other embodiments may be adapted and/or configured for use in other anatomies. It is to be understood that a broad variety of materials, dimensions and structures can be used to construct suitable embodiments, depending on the desired characteristics. The following examples of some embodiments are included by way of example only, and are not intended to be limiting.

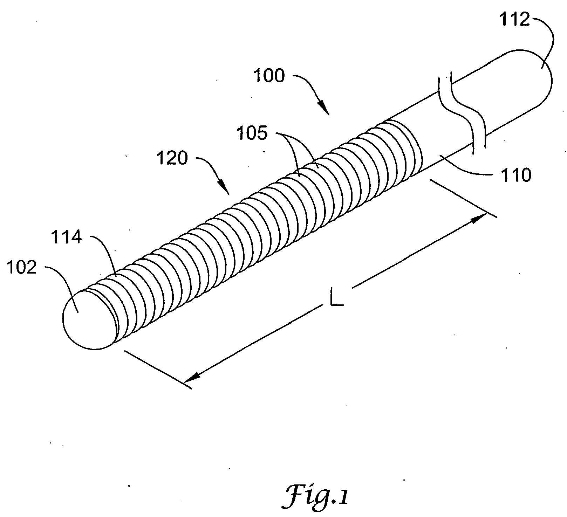

Figure 1 is a perspective view of an example coil 120 incorporated into an elongate intracorporeal or medical device 100. The elongate medical device 100 may include an elongate shaft or core 110. The elongate shaft or core 110 can have a proximal portion 112 and an opposing distal portion 114. The coil 120 (described below) can be disposed on a portion of the elongate shaft 110, for example, at the distal portion 114. A distal tip 102 can be disposed on an end of the coil 120 and/or the elongate shaft or core 110. The coil 120 can be wrapped in a helical fashion by conventional winding techniques. The pitch of adjacent turns 105 of the coil 120 may be tightly wrapped so that each turn touches the succeeding turn or the pitch may be set such that the coil 120 is wrapped in an open fashion. The coil 120 can be wound in a direction, such as clockwise or counterclockwise, that form a plurality of windings 105 a longitudinal distance denoted as a coil length L.

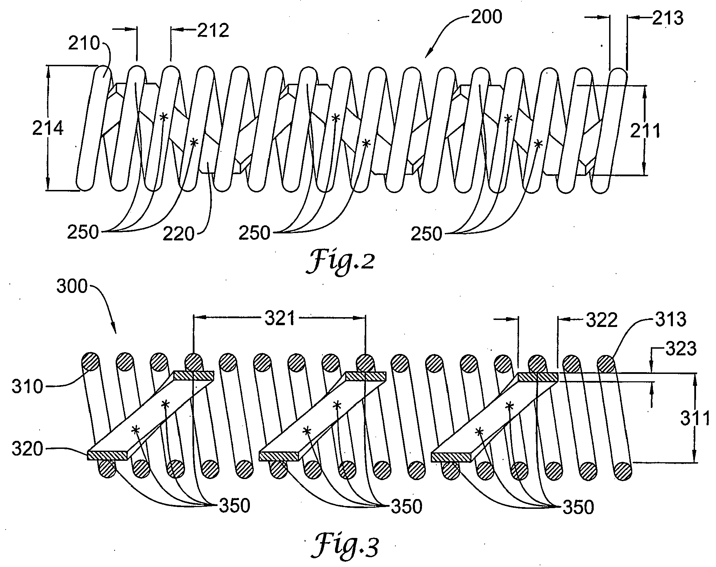

Figure 2 is a side elevation view of an example coil 200. Figure 3 is a cross-sectional view of an example coil 300 shown in Figure 2. The coil 200, 300 is formed with an outer coil 210, 310 disposed around one or more inner coils, such as inner coil 220, 320. The outer coil 210, 310 has a lumen 211, 311 defined by the inner diameter of the outer coil 210, 310. The inner coil 220, 320 can be coaxially disposed within the outer coil lumen 211, 311. The outer coil 210, 310 can be in contact with the inner coil 220, 320. The inner coil 220, 320 can have a length that is equal to or less than the length of the outer coil 210, 310. In some embodiments, however, the inner coil 220, 320 may have a length that is greater than the length of the outer coil 210, 310.

The outer coil 210, 310 and inner coil 220, 320 can be formed of a filament, such as a round wire or flat ribbon ranging in dimensions to achieve the desired flexibility. It can also be appreciated that other cross-sectional shapes or combinations of shapes may be utilized. For example, the cross-sectional shape of wires, filaments, or ribbons used to make the outer coil 210, 310 and inner coil 220, 320 may be oval, rectangular, square, triangular, polygonal, and the like, or any suitable shape.

The outer coil 210, 310 can be formed of a wire 213, 313 having a round cross-section. The wire 213, 313 can be any suitable diameter such as, for example, in the range of about 0.01 mm (0.0005 inch) to 0.25 mm (0.01 inch) or 0.025 mm (0.001 inch) to 0.1 mm (0.005 inch) or 0.05 mm or 0.002 inch. The outer diameter 214 of the outer coil 210, 310 can be any suitable diameter such as, for example, in the range of about 0.1 mm (0.005 inch) to 7.6 mm (0.3 inch) or 0.25 mm (0.01 inch) to 0.5 mm (0.02 inch or 3.8 mm (0.015 inch). The outer coil 210, 310 can be formed by winding the wire 213, 313 in a first direction, such as clockwise or counterclockwise. The outer coil. 210, 310 can have any suitable pitch 212 such as for example, in the range of about 0.025 mm (0.001 inch) to 0.25 mm (0.01 inch) or 0,05 mm (0.002 inch) to 0.2 mm (0.008 inch) or 0.1 mm (0.004 inch).

The inner coil 220, 320 can be formed of a ribbon or multiple ribbons having a flat or rectangular cross-section. The ribbon can be any suitable width 322 such as, for example, in the range of about 0.01 mm (0.0005 inch) to 0.25 mm (0.01 inch) or 0.025 mm (0.001 inch) to 0.1 mm (0.005 inch) or 0.08 mm (0.003 inch). The ribbon can be any suitable thickness 323 such as, for example, in the range of about 0.01 mm (0.0005 inch) to 0.25 mm (0.01 inch) or 0.01 mm (0.0005 inch) to 0.1 mm (0.005 inch) or 0.025 mm (0.001 inch). The outer diameter of the inner coil 220 320 can be any suitable size such as, for example, in the range of about 0.1 mm (0.005 inch) to 7.6 mm (0.3 inch) or 0.25 mm (0.01 inch) to 0.5 mm (0.02 inch) or 0.025 mm (0.01 inch). The inner coil 220, 320 can be formed by winding the wire in a second direction, such as clockwise or counterclockwise, which is a direction opposite of the first direction winding of the outer coil 210, 310. In some embodiments wherein the inner coil 220, 320 may be formed of multiple ribbons or filaments, the multiple ribbons or filaments may be wound in a second direction opposite the first direction winding of the outer coil 210, 310. In other embodiments wherein the inner coil 220, 320 may be formed of multiple ribbons or filaments, one or more ribbons or filaments may be wound in a first direction, similar to the direction of the outer coil 210, 310, and one or more ribbons or filaments may be wound in a second direction opposite the first direction winding of the outer coil 210, 310. The one or more filaments of the inner coil 220, 320 can have any suitable pitch 321 such as for example, in the range of about 0.13 mm (0.005 inch) to 2.5 mm (0.1 inch) or 0.25 mm (0.01 inch) to 1.3 mm (0.05 inch) or 0.5 mm (0.02 inch). In some embodiments, the inner coil 220, 320 pitch 321 can be greater than the outer coil 210, 310 pitch 212. For example, the inner coil 220, 320 pitch 321 can be in the range of about 1, 2, 3, 4, 5, 6, 7, 8, 9, or 10 times greater than the outer coil 210, 310 pitch 212.

The outer coil 210, 310 and inner coil 220, 320 can be formed of a variety of materials including metals, metal alloys, polymers, and the like. The outer coil 210, 310 and inner coil 220, 320 can be formed of the same or different material. Some examples of material for use in the coils 210, 310, 220, 320 include a metal or a metal alloy such as a stainless steel, for example 304V, 304L, and 316L stainless steel; alloys including nickel-titanium alloy such as linear elastic or superelastic (i.e. pseudoelastic) nitinol; nickel-chromium alloy; nickel-chromium-iron alloy; cobalt alloy; tungsten or tungsten alloys; MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si); hastelloy; monel 400; inconel 625; or the like; or other suitable material, or combinations or alloys thereof. Some additional examples of suitable material include a polymer material, such as a high performance polymer.

The outer coil 210, 310 and inner coil 220, 320 can be formed of or portions thereof can be made of, or coated or plated with, or otherwise include a radiopaque material. Radiopaque materials are understood to be materials capable of producing a relatively bright image on a fluoroscopy screen or another imaging technique during a medical procedure. This relatively bright image aids the user of medical device 100 in determining its location during a medical procedure. Some examples of radiopaque materials can include, but are not limited to, gold, platinum, palladium, tantalum, tungsten alloy, polymer material loaded with a radiopaque filler, and the like, or combinations or alloys thereof.

Additionally, the outer coil 210, 310 and inner coil 220, 320 can be formed of, or other portions of the device 100, can include materials or structure to impart a degree of MRI compatibility. For example, to enhance compatibility with Magnetic Resonance Imaging (MRI) machines, it may be desirable to make the outer coil 210, 310 and inner coil 220, 320, or other portions of the medical device 100, in a manner that would impart a degree of MRI compatibility. For example, the elongate shaft or core 110, the coil 120, 200, 300 or portions thereof, or other portions of the device 100, may be made of a material that does not substantially distort the image and create substantial artifacts (artifacts are gaps in the image). Certain ferromagnetic materials, for example, may not be suitable because they may create artifacts in an MRI image. The elongate shaft or core 110, the coil 120, 200, 300 or portions thereof, may also be made from a material that the MRI machine can image. Some materials that exhibit these characteristics include, for example, tungsten, Elgiloy, MP35N, nitinol, and the like, and others, or combinations or alloys thereof.

In some embodiments, the coil 120, 200, 300 can be made of a material that is compatible with the core wire 110 and the distal tip 102. The particular material used can be chosen in part based on the desired flexibility requirements or other desired characteristics. In some particular embodiments, the coil 120, 200, 300 can be formed from a superelastic or linear elastic nickel-titanium alloy, for example, linear elastic or superelastic nitinol.

The word nitinol was coined by a group of researchers at the United States Naval Ordinance Laboratory (NOL) who were the first to observe the shape memory behavior of this material. The word nitinol is an acronym including the chemical symbol for nickel (Ni), the chemical symbol for titanium (Ti), and an acronym identifying the Naval Ordinance Laboratory (NOL). Within the family of commercially available nitinol alloys, is a category designated "super elastic" (i.e. pseudoelastic) and a category designated "linear elastic". Although these two categories of material are similar in chemistry, they each exhibit distinct and useful mechanical properties. Either, or both superelastic and linear elastic nitinol can be used.

One example of a suitable nickel-titanium alloy that may exhibit linear elastic properties is FHP-NT alloy commercially available from Furukawa Techno Material Co. of Kanagawa, Japan. Some examples of suitable nickel-titanium alloys that may exhibit linear elastic characteristics include those disclosed in U.S. Patent Nos. 5,238,004 and 6,508,803.

The inner coil 220, 320 is affixed to the outer coil 210, 310 with a plurality of affixation points 250, 350 disposed along the coil length L. The affixation points 250, 350 couple the inner coil 220, 320 to the outer coil 210, 310 where the inner coil 220, 320 windings intersect or cross with the outer coil 210, 310 windings. There may be 2, 3, 4, 5, 6, 7, 8, 9, 10, 11, 12, 13, 14, 15, 16, 17, 18, 19, 20, 25, 30, 35, 40, 50, 100, 200 or more affixation points 250, 350 disposed in a uniform or non-uniform pattern along the coil length at the inner coil 220, 320 winding and outer coil 210, 310 winding intersections. For example, the affixation points 250, 350 may be disposed in a uniform density pattern along the coil length such that the properties of the coil assembly along the length thereof are generally uniform. In other examples, the density of affixation points 250, 350 along the coil length may vary to achieve desired characteristics. For example, in some embodiments, the density of affixation points (i.e. number of affixation points in a given length) in a distal portion of the coil length may be less than the density of affixation points in a proximal portion of the coil length, or vice versa. In some embodiments, there may be no affixation points along a portion of the coil length. In at least some embodiments, the portion having a lower density of, or an absence of, affixation points may provide for desirable flexibility characteristics. It should be understood that these configurations are given by way of example only, and that the density of affixation points along the coil length may vary as desired. In some embodiments, the affixation points 250, 350 can be along a plurality, a majority, or substantially all of the inner coil 220, 320 winding and outer coil 210, 310 winding intersections. For example, in some embodiments, affixation points may be disposed at 25% or more, 50% or more, 75% or more, or 90% or more of the winding intersections.

In at least some embodiments, the affixation points 250, 350 may only function to affix an inner coil 220, 320 winding to an outer coil 210, 310 winding together. For example, in at least some embodiments, the affixation points 250, 350 join an inner coil 220, 320 and an outer coil 210, 310 winding together, but do not act to join any other structure within the device 100. For example, in some such embodiments, the affixation points 250, 350 do not join the coil 120, 200, 300 to the shaft or core 110.

The affixation points 250, 350 by interconnecting the inner coil 220, 320 windings to the outer coil 210, 310 windings, can provide enhanced torque transmission along the coil length L and/or enhanced push-ability while still providing desired flexibility. The degree of enhanced torque transmission and/or push-ability is in part dependent on the number of affixation points 250, 350 along the length of the coil. The number of affixation points 250, 350 can be varied to obtain the desired characteristics.

The affixation points 250, 350 can be formed in any suitable manner, including for example welding, soldering, brazing, adhesive bonding, mechanical interlocking and the like. It is to be appreciated that various welding processes can be utilized without deviating from the scope of the invention. In general, welding refers to a process in which two materials, such as metals, metal alloys, or polymers are joined together by heating the two materials sufficiently to at least partially melt adjoining surfaces of each material so that they fuse to a relatively permanent union upon coupling. A variety of heat sources can be used to melt the adjoining materials. Examples of welding processes that can be suitable in some embodiments include LASER welding, resistance welding, TIG welding, micro plasma welding, electron beam, RF welding, and friction or inertia welding. In LASER welding, a light beam is used to supply the necessary heat. Laser welding can be beneficial in the processes contemplated by the invention, as the use of a laser light heat source can provide pinpoint accuracy. Additionally, laser energy can be used in other affixation techniques, such as soldering, brazing, or the like. LASER welding equipment that may be suitable in some embodiments is commercially available from Unitek Miyachi of Monrovia, California and Rofin-Sinar Incorporated of Plymouth, Michigan. Resistance welding equipment that may be useful in some embodiments is commercially available from Palomar Products Incorporated of Carlsbad, California and Polaris Electronics of Olathe, Kansas. TIG welding equipment that may be useful in some embodiments is commercially available from Weldlogic Incorporated of Newbury Park, California. Microplasma welding equipment that may be useful in some embodiments is commercially available from Process Welding Systems Incorporated of Smyrna, Tennessee.

In some embodiments, the outer coil 210, 310 and/or the inner coil 220, 320 can be pre-coated, or otherwise include an attachment material which can be thereafter activated to achieve affixation. For example, the outer coil 210, 310, and/or the inner coil 220, 320, or both, may be pre-coated with a heat activated binding material such as a solder or adhesive. For example, in the context of solder material, this is often referred to as "pre-tinning". The coils 210/220 or 310/320 can then be placed in the desired positions, and a suitable heat source, such as LASER, IR, radient, or other, can be used to activate the binding material.

The affixation points 250, 350 can be created or disposed on the inner coil 220, 320 winding and outer coil 210, 310 winding intersections prior to the attachment of the coil 120, 200, 300 to the structure of the device 100, or in some embodiments, can be created or disposed on the inner coil 220, 320 winding and outer coil 210, 310 winding intersections after attachment of the coil 120, 200, 300 to the structure of the device 100, such as the core or shaft 110 or the distal tip 102.

Such a coil assembly 120, 200, 300 including affixation points 250, 350, as discussed above, can be incorporated into a broad variety of medical devices. For example, as shown in Figure 1, the coil 120, 200, 300 can be incorporated into an elongate medical device 100, such as a guidewire, that may include an elongate shaft or core 110. The coil 120, 200, 300 can be disposed on a portion of the elongate shaft, for example, at the distal end 114. It should be understood, however, that such a coil 120, 200, 300 can be incorporated into a broad variety of medical devices.

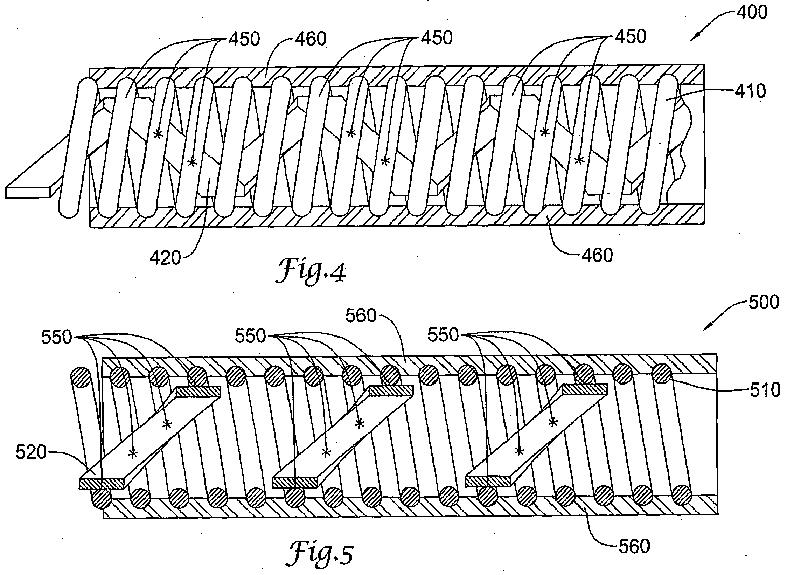

Figure 4 is a side elevation view of an example coil. Figure 5 is a cross-sectional view of an example coil shown in Figure 4. An outer coil 410, 510 is disposed about an inner coil 420, 520 as described above. The outer coil 410, 510 windings are affixed to the inner coil 420, 520 windings with a plurality of affixation points 450, 550 as described above. An outer member, such as an outer tubular member 460, 560 can be disposed about at least a portion of the outer coil 410, 510. The outer member 460, 560 can be formed from a variety of materials including metals or polymeric materials.

Some suitable materials to form the outer member 460, 560 include polymers, and like material. Examples of suitable polymer material include any of a broad variety of polymers generally known for use as guidewire outer member 460, 560. In some embodiments, the polymer material used is a thermoplastic polymer material. Some examples of some suitable materials include polyurethane, elastomeric polyamides, block polyamide/ethers (such as Pebax), silicones, and co-polymers. The outer member 460, 560 may be a single polymer, multiple layers, or a blend of polymers. By employing selection of materials and processing techniques, thermoplastic, solvent soluble and thermosetting variants of these materials can be employed to achieve the desired results. The outer member 460, 560 can be a hydrophilic or hydrophobic coating.

Hydrophillic polymer coatings include, for example, polyarylene oxides, polyvinylpyrolidones, polyvinylalcohols, hydroxyl alkyl cellulosics, aligns, saccarides, caprolactomes, and the like. Further examples of suitable polymeric materials for forming the outer tubular member 460, 560 include but are not limited to poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D, L-lactide-co-glycolide) (PLA/PGA), poly(glycolide-co-trimethylene carbonate) (PGA/PTMC), polyethylene oxide (PEO), polydioxanone (PDS), polycaprolactone (PCL), polyhydroxylbutyrate (PHBT), poly(phosphazene), poly D,L-lactide-co-caprolactone) (PLA/PCL), poly(glycolide-co-caprolactone) (PGA/PCL), polyanhydrides (PAN), poly(ortho esters), poly(phosphate ester), poly(amino acid), poly(hydroxy butyrate), polyacrylate, polyacrylamid, poly(hydroxyethyl methacrylate), polyurethane, polysiloxane and their copolymers.

Hydrophobic coatings such as fluoropolymers provide a dry lubricity which improves guide wire handling and device exchanges. Lubricious coatings improve steer-ability and improve lesion crossing capability. Suitable lubricious polymers are well known in the art and may include hydrophilic polymers such as polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. In some embodiments, the outer member 460, 560 is formed of a fluoropolymer such as, for example, polytetrafluoroethylene (PTFE).

In some embodiments, the outer member 460, 560 can aid in maintaining a constant outer diameter, may increase resistance to prolapse, and/or may effect characteristics of the coil or medical device, such as flexibility, column strength, lubricity, traction, tortion, torque response, or other such characteristics.

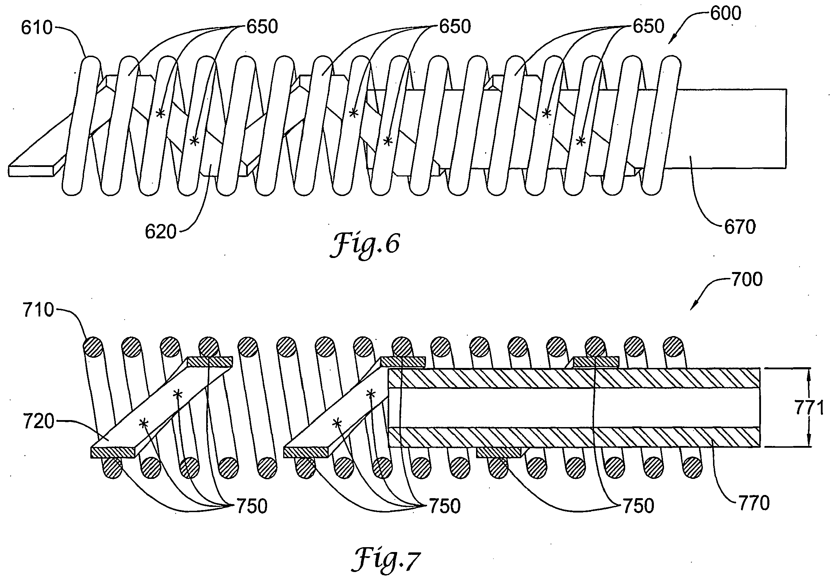

Figure 6 is a side elevation view of an example coil. Figure 7 is a cross-sectional view of an example coil shown in Figure 6. An outer coil 610, 710 is disposed about an inner coil 620, 720 as described above. The outer coil 610, 710 windings are affixed to the inner coil 620, 720 windings with a plurality of affixation points 650, 750 as described above. An inner member 670, 770 can be disposed within the inner coil 620, 720. The inner member 670, 770 can have an outer diameter suitable for disposal within the inner coil 620, 720 and to achieve desired characteristics of the coil, such as flexibility, support, and the like. In some embodiments, the inner member can have an outer diameter in the range of about 0.025 mm (0.001 inch) to 0.25 mm (0.01 inch) or 0.1 mm (0.005 inch) to 0.2 mm (0.008 inch). The inner member 670, 770 can be formed from a variety of materials including metals or polymeric materials. Some examples of polymeric materials useful for forming the inner member 670, 770 are described above. Some examples of metals useful for forming the inner member 670, 770 are also described above. The inner member 670, 770 may be solid in cross section, or may be tubular, defining a lumen, as shown.

In some embodiments, the inner member 670, 770, can aid in supporting the coil 600, 700, and/or may effect characteristics of the coil 600, 700 or medical device, such as flexibility, stiffness, or other characteristics. Additionally, in some embodiments, the inner member 670, 770 may be useful in construction of the coil, for example in maintaining alignment of coils 610, 710, and 620, 720.

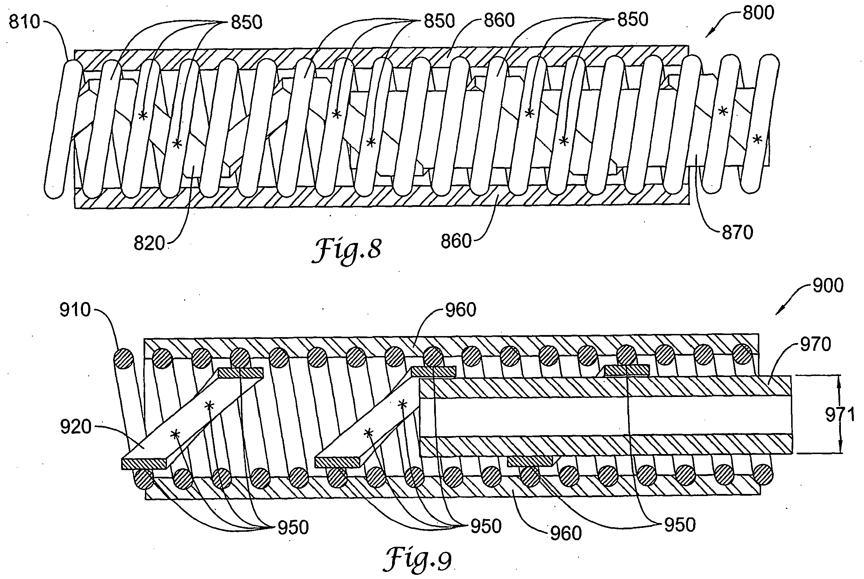

Figure 8 is a side elevation view of an example coil. Figure 9 is a cross-sectional view of an example coil shown in Figure 8. An outer coil 810, 910 is disposed about an inner coil 820, 920 as described above. The outer coil 810, 910 windings are affixed to the inner coil 820, 920 windings with a plurality of affixation points 850, 950 as described above. An outer tubular member 860, 960 is disposed about at least a portion of the outer coil 810, 910 as described above. An inner tubular member 870, 970 is disposed within the inner coil 820, 920 as described above.

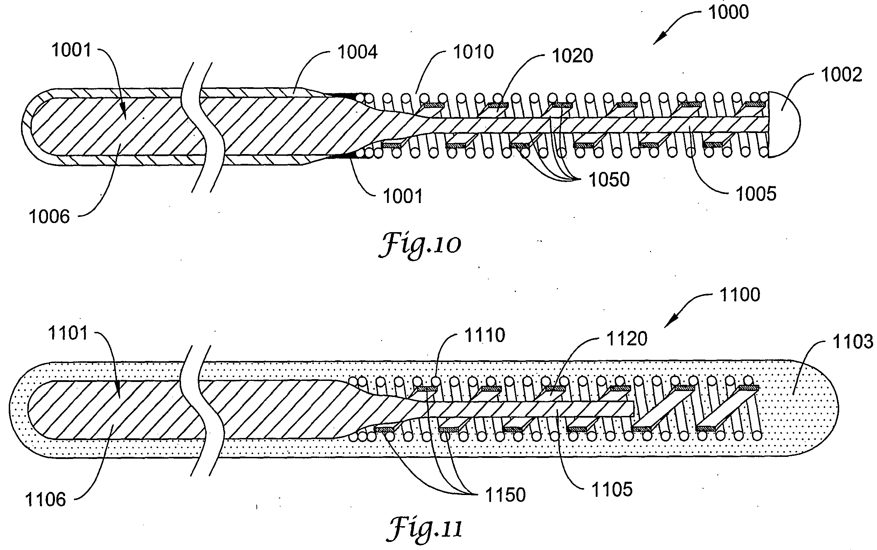

Figure 10 is a cross-sectional view of an alternative example of a guidewire 1000 with an example coil described above. The guidewire 1000 includes a core 1001. The core 1001 may have a proximal section 1006 and an opposing distal section 1005. The core 1001 may be sized and/or tapered to achieve desired characteristics, for example, flexibility, stiffness, or the like. For example, the distal section 1005 may include one or a series of taper and constant diameter sections as illustrated in Figure 10. In other embodiments, the proximal section 1006 may also include a series of taper and constant diameter sections. The tapered regions may be linearly tapered, tapered in a curvilinear fashion, uniformly tapered, non-uniformly tapered, or tapered in a step-wise fashion. The angle of any such tapers can vary, depending upon the desired flexibility characteristics. The length of the taper may be selected to obtain a more (longer length) or less (shorter length) gradual transition in stiffness. It can be appreciated that any portion of guidewire 1000 and/or guidewire sections 1005/1006 may be tapered and the taper can be in either the proximal or the distal direction. In some other embodiments, a guidewire 1000 core wire 1001 can have a profile in which the core wire has a greater number of constant diameter sections, separated by a greater number of taper sections. A guidewire 1000 core wire 1001 can have fewer or no tapers. The tapers can be as illustrated in Figure 10, or they can be longer (more gradual), or shorter (less gradual).

The tapered and constant diameter portions of the tapered region may be formed by any one of a number of different techniques, for example, by centerless grinding methods, stamping methods, and the like. The centerless grinding technique may utilize an indexing system employing sensors (e.g., optical/reflective, magnetic) to avoid excessive grinding of the connection. In addition, the centerless grinding technique may utilize a CBN or diamond abrasive grinding wheel that is well shaped and dressed to avoid grabbing the core wire during the grinding process. Some examples of suitable grinding methods are disclosed in U.S. Patent Application No. 10/346,698 (Pub. No. U.S. 2004/0142643). The narrowing and constant diameter portions as shown in Figure 10 are not intended to be limiting. One of skill will recognize that a guidewire 1000 core wire 1001 can have a profile different from that illustrated in Figure 10.

With reference to the embodiment shown in Figure 10, the elongate shaft or core 1001 can have a solid cross-section or a hollow cross-section. In other embodiments, the elongate shaft or core 1001 can include a combination of areas having solid cross-sections and hollow cross sections. Moreover, the elongate shaft or core 1001 can be made of rounded wire, flattened ribbon, or other such structures having various cross-sectional geometries. The cross-sectional geometries along the length of the elongate shaft or core 1001 can also be constant or can vary. For example, Figure 1 depicts the elongate shaft or core 110 as having a generally round cross-sectional shape. It can be appreciated that other cross-sectional shapes or combinations of shapes may be utilized. For example, the cross-sectional shape of the elongate shaft or core 110, 1001 may be oval, rectangular, square, polygonal, and the like, or any suitable shape.

In some embodiments, the elongate shaft or core 1001 can be formed of any suitable metallic, polymeric or composite material. In some embodiments, part or all of the elongate shaft or core 1001 can be formed of a metal or a metal alloy such as a stainless steel, such as 304V, 304L, and 316L stainless steel; alloys including nickel-titanium alloy such as linear elastic or superelastic (i.e. pseudoelastic) nitinol; nickel-chromium alloy; nickel-chromium-iron alloy; cobalt alloy; tungsten or tungsten alloys; MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si); hastelloy; monel 400; inconel 625; or the like; or other suitable material, or combinations or alloys thereof. The particular material used can be chosen in part based on the desired flexibility requirements or other desired characteristics of the elongate shaft or core 1001. In some particular embodiments, the elongate shaft or core 1001 can be formed from a superelastic or linear elastic nickel-titanium alloy, for example, linear elastic or superelastic nitinol, for example, those discussed above with regard to the coil 120, 200, 300.

The entire elongate shaft or core 1001 can be made of the same material, or in some embodiments, can include portions or sections that are made of different materials. In some embodiments, the material used to construct different portions of the core wire 1001 can be chosen to impart varying flexibility and stiffness characteristics to different portions of the wire. For example, a proximal portion 1006 and a distal portion 1005 can be formed of different materials (i.e., materials having different moduli of elasticity) resulting in a difference in flexibility. In some embodiments, the material used to construct the proximal portion 1006 can be relatively stiff for push-ability and torque-ability, and the material used to construct the distal portion 1005 can be relatively flexible by comparison for better lateral track-ability and steer-ability. For example, the proximal portion 1006 can be formed of, for example, straightened 304v stainless steel wire, and the distal portion 1005 can be formed of, for example, a straightened super elastic or linear elastic alloy (e.g., nickel-titanium) wire.

In embodiments where different portions of elongate shaft or core 1001 are made of different material, the different portions can be connected using any suitable connecting techniques. For example, the different portions of the elongate shaft or core 1001 can be connected using welding, soldering, brazing, adhesive, or the like, or combinations thereof. Additionally, some embodiments can include one or more mechanical connectors or connector assemblies to connect the different portions of the elongate shaft or core 1001 that are made of different materials. The connector may comprise any structure generally suitable for connecting portions of an elongate shaft or core 1001. One example of a suitable structure includes a structure such as a hypotube or a coiled wire which has an inside diameter sized appropriately to receive and connect the different portions of the elongate shaft or core 1001. Some methods and structures that can be used to construct medical devices are disclosed in U.S. Patent No. 6,918,882, and U.S. Patent Application Ser. Nos. 10/086,992 (Pub. No. U.S. 2003/0069521), and 10/376,068 (Pub. No. U.S. 2004/0167442).

Additionally, the structure used to construct the core wire 1001 can be designed such that a proximal portion 1006 is relatively stiff for push-ability and torque-ability, and distal portion 1005 is relatively flexible by comparison for better lateral track-ability and steer-ability. For example, in some embodiments, a proximal portion 1006 has a constant or generally uniform diameter along its length to enhance stiffness. However, embodiments including a proximal portion 1006 having a tapered portion or a series of tapered portions are also contemplated. The diameter of the proximal portion 1006 can be sized appropriately for the desired stiffness characteristics dependent upon the material used. For example, in some embodiments, a proximal portion 1006 can have a diameter in the range of about 0.25 to about 0.64 mm (0.010 to about 0.025 inches) or greater, and in some embodiments, in the range of about 0.25 to about 0.46 mm (0.010 to about 0.018 inches) or greater.

A distal portion 1005 can likewise be constant diameter, can be continuously tapered, or can have a tapered section or a number or a series of tapered sections of differing diameters. In embodiments where the structure of core wire 1001 is designed such that a distal portion 1005 is relatively flexible by comparison to the proximal portion 1006, the distal portion 1005 can include at least one tapered or reduced diameter portion for better flexibility characteristics.

The lengths of the proximal portion 1006 and distal portion 1005 are typically, but not always dictated by the length and flexibility characteristics desired in the final medical device. In some embodiments, the proximal portion 1006 can have a length in the range of about 50 to about 300 centimeters, and the distal portion 1005 can have a length in the range of about 3 to about 50 centimeters.

The outer coil 1010 and inner coil 1020 can be disposed about a portion of the core, for example, the tapered distal section 1005 as discussed above. It should be understood, however, that the coils 1010, 1020 can be disposed about other portions of the core, as desired. The outer coil 1010 is fixed to the inner coil 1020 with a plurality of affixation points 1050 as described above. The core 1001 can be formed from a variety of materials as described above. The outer coil 1010, the inner coil 1020, and/or both may be attached to the core 1001 at desired attachment positions and in any suitable manner, for example soldering, brazing, welding, adhesive bonding, friction fitting, mechanical attachment, the use of additional connector structures, or the like. For example, in some embodiments, the outer coil 1010, the inner coil 1020, and/or both may be attached to the core 1001 at an attachment point adjacent the proximal ends thereof, and at an attachment point adjacent the distal ends thereof. However, it should be understood that other attachment points, such as intermediate attachment points, may be used. Because the inner coil 1020 and outer coil 1010 are attached to one another, in at least some embodiments, only one of the inner coil 1010 or outer coil 1020 need be attached directly to the core 1001. For example, in some embodiments, only the outer coil 1010 may be attached to the core 1001, while the inner coil 1020 is attached only to the outer coil 1010 at one or more attachment points, and is essentially free of any other direct connection to a core 1001, or in some cases, is free of direct connection to any other structure in the device other than the outer coil 1010. Similarly, in other embodiments, only the inner coil 1020 may be attached to the core 1001, while the outer coil 1010 is attached only to the inner coil 1020 at one or more attachment points, and is essentially free of any other direct connection to a core, or in some cases, is free of direct connection to any other structure in the device other than the inner coil 1020.

A guidewire 1000 in accordance with some embodiments of the invention can optionally include a coating layer 1004 such as a lubricious coating layer over part or all of the guidewire assembly 1000 or even over part of it. Hydrophobic coatings such as fluoropolymers provide a dry lubricity which improves guide wire handling and device exchanges. Lubricious coatings improve steer-ability and improve lesion crossing capability. Suitable lubricious polymers are well known in the art and may include hydrophilic polymers such as polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. In some embodiments, the more distal portion 1005 of the guidewire 1000 is coated with a hydrophilic polymer and the more proximal portion 1006 of the guidewire 1000 is coated with a fluoropolymer 1004, such as polytetrafluoroethylene (PTFE).

In at least some embodiments, portions or all of the elongate shaft or core 1001, the outer coil 1010 and/or inner coil 1020, or other structures included within the guidewire 1000 may also be doped with, coated or plated with, made of, or otherwise include a radiopaque material. Additionally, in some embodiments, a degree of MRI compatibility can be imparted into the guidewire 1000, as discussed above.

The distal tip 1002 can be formed from a variety of different materials, depending on desired performance characteristics. In some embodiments, the distal tip 1002 can form an atraumatic portion on the distal end of the device 1000. In some embodiments, the distal tip 1002 can be formed of a material such as a metallic material that is amenable to being welded, soldered, or otherwise attached to the distal end 1005 of the elongate shaft or core 1001. For example, in some embodiments, the distal tip 1002 can be a solder tip that is disposed via soldering at the distal end of the device and forms an atraumatic rounded portion. In other embodiments, the distal tip can be prefabricated, or partially prefabricated, structure that is thereafter attached to the distal end of the device using suitable attachment techniques, such as welding, soldering, brazing, crimping, friction fitting, adhesive bonding, mechanical interlocking and the like. A variety of different processes, such as soldering, deep drawing, roll forming or metal stamping, metal injection molding, casting and the like, can be used to form the distal tip 1002.

In some embodiments, laser or plasma welding can be used to secure the distal tip 1002, the outer coil 1010 and/or inner coil 1020 and the elongate shaft or core 1001 securely together. In laser welding, a light beam is used to supply the necessary heat. Laser welding can be beneficial in the processes contemplated by the invention, as the use of a laser light heat source can provide pinpoint accuracy. In some embodiments, laser diode soldering can be useful.

In some embodiments, it may be beneficial, but not always necessary, that the distal tip 1002 be formed of a material that is compatible with the particular joining technique used to connect the tip 1002 to the other structure. For example, in some particular embodiments, it can be beneficial but not necessary for the distal tip 1002 to be formed of the same metal or metal alloy as the distal end 1005 of the elongate shaft or core 1001. For example, if the elongate shaft or core 1001 is formed of stainless steel, it can be beneficial for the distal tip 1002 to be formed of stainless steel. In other embodiments, both of the distal tip 1002 and the distal end 1005 of the elongate shaft or core 1001 can be formed of the same metal alloy, such as nitinol.

To form the assembly 1000 shown in Figure 10, the outer coil 1010 and inner coil 1020 can be positioned proximate the elongate shaft or core 1001 as illustrated. The outer coil 1010 and/or inner coil 1020 and/or both can be secured to the elongate shaft or core 1001 in any suitable manner, including for example welding, soldering, brazing, crimping, friction fitting, adhesive bonding, mechanical interlocking and the like. In the embodiment shown, the outer coil 1010 and/or inner coil 1020 and/or both can be secured at its proximal end to the elongate shaft or core 1001 at a proximal attachment point 1011, and can be secured at its distal end to the elongate shaft or core 1001 via the distal tip 1002. In some embodiments, the distal tip 1002 is a solder tip or a weld tip that is soldered or welded to the elongate shaft or core 1001 and the outer coil 1010 and/or inner coil 1020, and/or both, and forms an atraumatic tip. In other embodiments, the distal tip 1002 is prefabricated, or partially prefabricated, and is connected to the elongate shaft or core 1001 and the outer coil 1010 and/or inner coil 1020 using a suitable attachment technique.

It should also be understood that the device 1000 can include additional structure, such as shaping ribbons, marker bands and/or coils, additional inner or outer coils, inner or outer sheaths, and the like. Those of skill in the art and others will recognize how to incorporate such additional structures into the device, as is generally known.

Figure 11 is a cross-sectional view of an alternative guidewire 1100 with a example coil as described above. The outer coil 1110 and inner coil 1120 are disposed over a portion of the core 1101 and a polymer sheath or sleeve 1103 is disposed over the core 1101 and outer coil 1110 and inner coil 1120. A plurality of affixation points 1150 join the inner coil 1120 to the outer coil 1110 at the coil winding intersections as described above. The inner and outer coils 1120/1110 and the core wire 1101 may include structure and materials as described in the embodiments discussed above.

In this embodiment a polymer tip guidewire 1100 is formed by including the polymer sheath or sleeve 1103 that forms a rounded tip over the outer coil 1110 and inner coil 1120. The polymer sheath or sleeve 1103 can be made from any material that can provide the desired strength, flexibility or other desired characteristics.

The use of a polymer can serve several functions, such as improving the flexibility properties of the guidewire assembly. Choice of polymers for the sheath or sleeve 1103 will vary the flexibility of the guidewire. For example, polymers with a low durometer or hardness will make a very flexible or floppy tip. Conversely, polymers with a high durometer will make a tip which is stiffer. The use of polymers for the sleeve can also provide a more atraumatic tip for the guidewire. An atraumatic tip is better suited for passing through fragile body passages. Finally, a polymer can act as a binder for radiopaque materials, as discussed in more detail below.

Some suitable materials to form the sleeve 1103 include polymers, and like material. Examples of suitable polymer material include any of a broad variety of polymers generally known for use as guidewire polymer sleeves 1103. In some embodiments, the polymer material used is a thermoplastic polymer material. Some examples of some suitable materials include polyurethane, elastomeric polyamides, block polyamide/ethers (such as Pebax), silicones, and co-polymers. The sleeve 1103 may be a single polymer, multiple layers, or a blend of polymers. By employing careful selection of materials and processing techniques, thermoplastic, solvent soluble and thermosetting variants of these materials can be employed to achieve the desired results.

Further examples of suitable polymeric materials for forming the sleeve 1103 include but are not limited to poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D, L-lactide-co-glycolide) (PLA/PGA), poly(glycolide-co-trimethylene carbonate) (PGA/PTMC), polyethylene oxide (PEO), polydioxanone (PDS), polycaprolactone (PCL), polyhydroxylbutyrate (PHBT), poly(phosphazene), poly D,L-lactide-co-caprolactone) (PLA/PCL), poly(glycolide-co-caprolactone) (PGA/PCL), polyanhydrides (PAN), poly(ortho esters), poly(phosphate ester), poly(amino acid), poly(hydroxy butyrate), polyacrylate, polyacrylamid, poly(hydroxyethyl methacrylate), polyurethane, polysiloxane and their copolymers.

In some embodiments, the sheath or sleeve 1103, or portions thereof, can include, or be doped with, radiopaque material to make the sheath or sleeve 1103, or portions thereof, more visible when using certain imaging techniques, for example, fluoroscopy techniques. Any suitable radiopaque material known in the art can be used. Some examples include precious metals, tungsten, barium subcarbonate powder, and the like, and mixtures thereof. In some embodiments, the polymer can include different sections having different amounts of loading with radiopaque material. For example, the sheath or sleeve 1103 can include a distal section having a higher level of radiopaque material loading, and a proximal section having a correspondingly lower level of loading.

In some embodiments, it is also contemplated that a separate radiopaque member or a series of radiopaque members, such as radiopaque coils, bands, tubes, or other such structures could be attached to the guidewire core wire 1101, or incorporated into the core wire by plating, drawing, forging, or ion implantation techniques.

The sheath or sleeve 1103 can be disposed around and attached to the guidewire assembly 1100 using any suitable technique for the particular material used. In some embodiments, the sheath or sleeve 1103 can be attached by heating a sleeve of polymer material to a temperature until it is reformed around the guidewire assembly 1100. In some other embodiments, the sheath or sleeve 1103 can be attached using heat shrinking techniques. In other embodiments, the sheath or sleeve 1103 can be extruded onto the guidewire. The sleeve 1103 can be finished, for example, by a centerless grinding or other method, to provide the desired diameter and to provide a smooth outer surface.

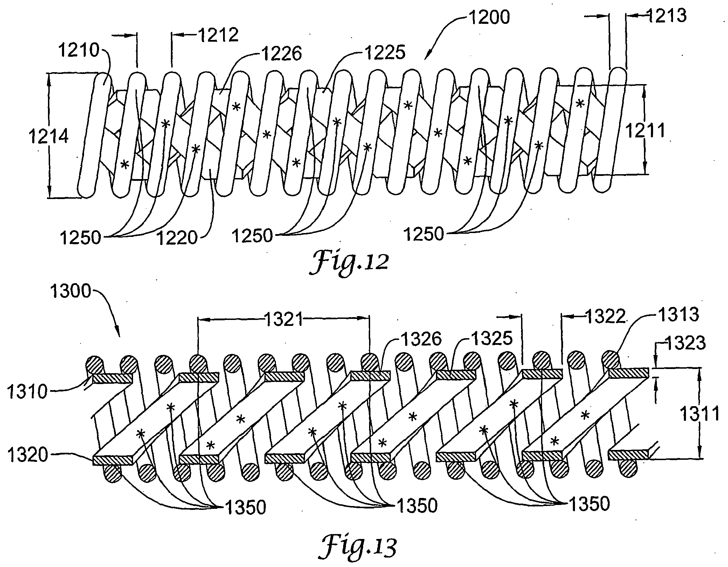

Figure 12 is a side elevation view of an example coil 1200. Figure 13 is a cross-sectional view of an example coil 1300 shown in Figure 12. The coil 1200, 1300 is formed with an outer coil 1210, 1310 disposed around an inner coil 1220, 1320 as described above. The inner coil 1220, 1320 may be positioned at least partially within the lumen 1211, 1311 of the outer coil 1210, 1310. The inner diameter of the outer coil 1210, 1310 may define the lumen 1211, 1311. In the embodiment shown, the inner coil 1220, 1320 is coaxially disposed in the lumen 1211, 1311 of the outer coil 1210, 1310. The outer coil 1210, 1310 may be in direct contact with and attached to the inner coil 1220, 1320 at points of intersection between the outer coil 1210, 1310 and inner coil 1220, 1320. The outer coil 1210, 1310 windings may be affixed to the inner coil 1220, 1320 windings with a plurality of affixation points 1250, 1350. Affixation points 1250, 1350 may be located at the points of intersection between the outer coil 1210, 1310 and inner coil 1220, 1320.

The outer coil 1210, 1310 may be formed of a filament, such as a wire 1213, 1313 having a round cross-section. The wire 1213, 1313 may be any suitable diameter as discussed above. The outer coil 1210, 1310 may be formed by winding the wire 1213, 1313 in a first direction, such as clockwise or counterclockwise. The outer coil 1210, 1310 may have any suitable pitch 1212 and any suitable outer diameter 1214 as discussed above.

The inner coil 1220, 1320 may be formed of a plurality of filaments, such as a first ribbon 1225, 1325 and a second ribbon 1226, 1326. The first and second ribbons 1225, 1325 and 1226, 1326 may have a flat or rectangular cross-section. The ribbons may have any suitable width 1322 and any suitable thickness 1323 as discussed above. The first ribbon 1225, 1325 may have dimensions similar to the second ribbon 1226, 1326, or the first ribbon 1225, 1325 may have dissimilar dimensions. Additionally, the outer diameter of the inner coil 1220, 1320 may be any suitable diameter.

As shown in Figures 12 and 13, the first and second ribbons 1225, 1325 and 1226, 1326 forming the inner coil 1220, 1320 may be helically wound in an open fashion. The first ribbon 1225, 1325 may be wound in a second direction, such as clockwise or counterclockwise, which is a direction opposite of the first direction winding of the outer coil 1210, 1310. The second ribbon 1226, 1326 may also be wound in a second direction, such as clockwise or counterclockwise, which is a direction opposite of the first direction winding of the outer coil 1210, 1310. Thus, the first ribbon 1225, 1325 and the second ribbon 1226, 1326 may be co-wound such that the windings of the first ribbon 1225, 1325 are interposed between the windings of the second ribbon 1226, 1326. In other words, the windings of the first ribbon 1225, 1325 may alternate with the windings of the second ribbon 1226, 1326 along the length of the inner coil 1220, 1320. The first ribbon 1225, 1325 may helically extend substantially parallel to the second ribbon 1226, 1326 along the length of the inner coil 1220, 1320. In the embodiment shown, the first ribbon 1225, 1325 may be radially opposed to the second ribbon 1226, 1326. Thus, at any cross-section taken along the length of the inner coil 1220, 1320, the first ribbon 1225, 1325 may be located at a position radially opposite the position of the second ribbon 1226, 1326. Although the inner coil 1220, 1320 is shown as including two ribbons 1225, 1325 and 1226, 1326 wound in a similar direction, additional embodiments of the inner coil 1220, 1320 may include additional ribbons and/or may include ribbons wound in a different or opposite direction. For example, the inner coil 1220, 1320 may include three or more filaments and/or the inner coil 1220, 1320 may include filaments, such as a first ribbon and a second ribbon wound in opposite directions.

Additional embodiments may include three or more coils forming a coil assembly. A third coil may be helically wound in a first direction, such as clockwise or counterclockwise, which is a direction similar to the first direction winding of the outer coil, or a third coil may be helically wound in a second direction, which is a direction opposite the first direction winding of the outer coil. Such embodiments may incorporate characteristics of one or more previous embodiments disclosed herein.

### EXAMPLE

A finite element analysis (FEA) was performed to compare certain properties of a prophetic coil as describe herein with the analogous properties of two comparative examples. The following is a prophetic example performed using FEA including select results compiled during the analysis.

An illustrative embodiment of a coil formed by winding an outer coil around an inner coil was analyzed. The inner coil was selected to comprise 304 stainless steel and included two ribbons of material wound in a first direction, with each inner coil ribbon wound at a pitch of 0.76 mm and spaced apart form one another an equal distance of about 0.38 mm. The width of each inner coil ribbon was set to about 0.1 mm and the thickness of each coil ribbon was set to about 0.05 mm. The outer diameter of the inner coil was set to about 0.25 mm.

An outer coil was wound about the inner coil in a second direction opposite the first direction. The outer coil was chosen to comprise 304 stainless steel. The outer coil had a diameter of about 0.05 mm and wound at a pitch of 0.075 mm. The outer coil had an outer diameter of about 0.33 mm and an inner diameter of about 0.25 mm. The outer coil was affixed to the inner coil at points where the inner coil contacted the outer coil. The outer coil was affixed to the inner coil via laser welding.

A first comparative example coil was formed by winding a wire about a solid core. Both the solid core and the wire was formed of 304 stainless steel. The solid core had a diameter of about 0.18 mm. The wire had a diameter of about 0.05 mm and was wound about the solid core at a pitch of about 0.1 mm. The wire was affixed to the solid core via laser welding.

A second comparative example was only a solid core. The solid core was formed of 304 stainless steel. The solid core had a diameter of about 0.18 mm.

A conventional finite element analysis for both torque and moment loads was preformed on a 1.27 mm length of the illustrative coil and 0.61 mm length of each comparative example. The solid core (second comparative example) had a (torsional rigidity)/(flexural rigidity) ratio of about 0.73. The threaded wire (first comparative example) had a (torsional rigidity)/(flexural rigidity) ratio of about 0.81. The illustrative example had a (torsional rigidity)/(flexural rigidity) ratio of about 1.97.

The present invention should not be considered limited to the particular examples described above, but rather should be understood to cover all aspects of the invention as fairly set out in the attached claims. Various modifications, equivalent processes, as well as numerous structures to which the present invention may be applicable will be readily apparent to those of skill in the art to which the present invention is directed upon review of the instant specification. It should be understood that this disclosure is, in many respects, only illustrative. Changes may be made in details, particularly in matters of shape, size, and arrangement of steps without exceeding the scope of the invention. The scope of the invention is, of course, defined in the language in which the appended claims are expressed.

## Claims

1. A coil assembly for use in a medical device, the coil assembly comprising:
a first coil member formed of a round wire wound in a first direction and defining a plurality of coil windings having an open pitch, the first coil member defining a lumen; and
a second coil member formed of a ribbon wound in a second direction different from the first direction and defining a plurality of coil windings having an open pitch, wherein a portion of the second coil member is disposed within the lumen of the first coil member such that coil windings of the first coil member intersect with coil windings of the second coil member to create a plurality of spaced coil intersection areas, and the first coil member is attached to the second coil member at a plurality of the intersection areas.

2. The coil assembly of claim 1, wherein the first coil is attached to the second coil at 25% or more of the coil intersection areas.

3. The coil assembly of claim 1, wherein the first coil is attached to the second coil at a majority of the coil intersection areas.

4. The coil assembly of claim 1, wherein the first coil is attached to the second coil at 75% or more of the coil intersection areas.

5. The coil assembly of claim 1, wherein the first coil is attached to the second coil at 90% or more of the coil intersection areas.

6. The coil assembly of claim 1, wherein the first coil is attached to the second coil at substantially all of the coil intersection areas.

7. The coil assembly of claim 1, furthercomprising an elongate shaft, wherein the first coil member is disposed about at least a portion of the elongate shaft.

8. The coil assembly according to claim 7, wherein the second coil member comprises a first filament and a second filament.

9. The coil assembly according to claim 8, wherein the first filament and the second filament are co-wound such that windings of the first filament are interposed between windings of the second filament.

10. The coil assembly according to claim 8 or 9, wherein the first filament and the second filament are radially opposed to one another.

11. The coil assembly according to any of claims 7-10, further comprising a third coil disposed within the first coil lumen.

12. The coil assembly according to claim 11, wherein the third coil is wrapped in the second direction.

13. The coil assembly according to any of claims 7-12, wherein the second coil member is formed of a flat ribbon.

14. The coil assembly according to any of claims 7-13, wherein the first coil member and second coil member both have an open coil pitch.

15. The coil assembly according to any of claims 7-14, wherein the first coil member has a first coil pitch and the second coil member has a second coil pitch that is greater than the first coil pitch.

16. The coil assembly of claim 1, further comprising a core wire having a tapered distal region, wherein the first coil member is disposed about at least a portion of the tapered distal region.

17. The coil assembly according to claim 16, further comprising an inner member disposed within the second coil member.

18. The coil assembly according to claim 17, wherein the inner member is a tubular member.

19. The coil assembly according to claim 17 or 18, wherein the inner member is polymeric.

20. The coil assembly according to any of claims 16-19, further comprising an outer member disposed about the first coil member.

21. The coil assembly according to claim 20, wherein the outer member is polymeric.

22. The coil assembly according to any of claims 16-21, wherein the first coil member has a first coil pitch and the second coil member has a second coil pitch that is five times greater than the first coil pitch.

23. The coil assembly according to any of claims 16-22, wherein the core wire further includes a proximal portion having an outer diameter and the first coil member has an outer diameter equal to the outer diameter of the proximal portion.

24. A method of making a coil assembly for use in a medical device, the method comprising:
providing a first coiled member formed of a round wire wound in a first direction and defining a plurality of coil windings having an open pitch, the first coil member defining a lumen;
providing a second coiled member formed of a ribbon wound in a second direction different from the first direction and defining a plurality of coil windings having an open pitch;
disposing a portion of the second coil member within the lumen of the first coil member such that coil windings of the first coil member intersect with coil windings of the second coil member to create a plurality of spaced coil intersection areas; and
attaching the first coil member to the second coil member at a plurality of the intersection areas.

25. The method of claim 24, wherein the first coil member is attached to the second coil member at 25% or more of the coil intersection areas.

26. The method of claim 24, wherein the first coil member is attached to the second coil member at a majority of the coil intersection areas.

27. The method of claim 24, wherein the first coil member is attached to the second coil member at 75% or more of the coil intersection areas.

28. The method of claim 24, wherein the first coil member is attached to the second coil member at 90% or more of the coil intersection areas.

29. The method of claim 24, wherein the first coil member is attached to the second coil member at substantially all of the coil intersection areas.

30. The method of claim 24, further comprising;
attaching an elongated shaft to the first or the second coil member, wherein the first coil member includes an outer coil and wherein the second coil member includes an inner coil.

31. The method according to claim 30, wherein disposing the outer coil around the inner coil comprises disposing the outer coil around and in direct contact with the inner coil.

32. The method according to claim 30 or 31, wherein the inner coil includes a first filament and a second filament.

33. The method according to claim 32, wherein the first filament and the second filament are co-wound such that windings of the first filament are interposed between windings of the second filament.

34. The method according to claim 32 or 33, wherein the first filament and the second filament are radially opposed to one another.

35. The method according to any of claims 30-34, wherein the affixing the inner coil to the outer coil comprises welding the inner coil to the outer coil.

36. The method according to claim 35, wherein welding the inner coil to the outer coil comprises laser welding the inner coil to the outer coil.

37. The method according to claim 35, wherein welding the inner coil to the outer coil comprises RF welding the inner coil to the outer coil.

38. The method according to any of claims 30-34, wherein affixing the inner coil to the outer coil comprises soldering the inner coil to the outer coil.

39. The method according to any of claims 30-34, wherein affixing the inner coil to the outer coil comprises bonding the inner coil to the outer coil with adhesive.

40. The method according to any of claims 30-39, wherein the inner coil has a first coil pitch and the outer coil has a second coil pitch that is less than the first coil pitch.

41. The method according to claim 40, wherein the first coil pitch is at least five times greater than the second coil pitch.

42. The method according to any of claims 30-41, wherein affixing the inner coil to the outer coil with a plurality of affixation points comprises, affixing the inner coil to the outer coil with 10 or more affixation points.

## Patentansprüche

1. Spulenanordnung zur Verwendung in einer medizinischen Vorrichtung, wobei die Spulenanordnung aufweist:
ein erstes Spulenelement, das aus einem runden Draht ausgebildet ist, der in eine erste Richtung gewickelt ist und mehrere Spulenwindungen definiert, die einen offenen Zwischenraum aufweisen, wobei das erste Spulenelement ein Lumen definiert; und
ein zweites Spulenelement, das aus einem Band ausgebildet ist, das in eine zweite, zur ersten Richtung unterschiedliche Richtung gewickelt ist und mehrere Spulenwindungen definiert, die einen offenen Zwischenraum aufweisen, wobei ein Abschnitt des zweiten Spulenelements im Lumen des ersten Spulenelements angeordnet ist, so dass sich Spulenwindungen des ersten Spulenelements mit Spulenwindungen des zweiten Spulenelements überschneiden, um mehrere beabstandete Spulenüberschneidungsbereiche zu erzeugen, und das erste Spulenelement am zweiten Spulenelement an mehreren der Überschneidungsbereiche befestigt ist.

2. Spulenanordnung nach Anspruch 1, wobei die erste Spule an der zweiten Spule an 25% oder mehr der Spulenüberschneidungsbereiche befestigt ist.

3. Spulenanordnung nach Anspruch 1, wobei die erste Spule an der zweiten Spule an einer Mehrzahl der Spulenüberschneidungsbereiche befestigt ist.

4. Spulenanordnung nach Anspruch 1, wobei die erste Spule an der zweiten Spule an 75% oder mehr der Spulenüberschneidungsbereiche befestigt ist.

5. Spulenanordnung nach Anspruch 1, wobei die erste Spule an der zweiten Spule an 90% oder mehr der Spulenüberschneidungsbereiche befestigt ist.

6. Spulenanordnung nach Anspruch 1, wobei die erste Spule an der zweiten Spule im Wesentlichen an allen Spulenüberschneidungsbereiche befestigt ist.

7. Spulenanordnung nach Anspruch 1, die ferner einen länglichen Schaft aufweist, wobei das erste Spulenelement um mindestens einen Abschnitt des länglichen Schafts angeordnet ist.

8. Spulenanordnung nach Anspruch 7, wobei das zweite Spulenelement ein erstes Filament und ein zweites Filament aufweist.

9. Spulenanordnung nach Anspruch 8, wobei das erste Filament und das zweite Filament gemeinsam gewickelt sind, so dass Windungen des ersten Filaments zwischen Windungen des zweiten Filaments angeordnet sind.

10. Spulenanordnung nach Anspruch 8 oder 9, wobei sich das erste Filament und das zweite Filament einander radial gegenüberliegen.

11. Spulenanordnung nach einem der Ansprüche 7 bis 10, die ferner eine dritte Spule aufweist, die im ersten Spulenlumen angeordnet ist.

12. Spulenanordnung nach Anspruch 11, wobei die dritte Spule in die zweite Richtung gewickelt ist.

13. Spulenanordnung nach einem der Ansprüche 7 bis 12, wobei das zweite Spulenelement aus einem flachen Band ausgebildet ist.

14. Spulenanordnung nach einem der Ansprüche 7 bis 13, wobei das erste Spulenelement und das zweite Spulenelement beide einen offenen Spulenzwischenraum aufweisen.

15. Spulenanordnung nach einem der Ansprüche 7 bis 14, wobei das erste Spulenelement einen ersten Spulenzwischenraum aufweist und das zweite Spulenelement einen zweiten Spulenzwischenraum aufweist, der größer als der erste Spulenzwischenraum ist.

16. Spulenanordnung nach Anspruch 1, die ferner einen Kerndraht aufweist, der einen verjüngten distalen Bereich aufweist, wobei das erste Spulenelement um mindestens einen Abschnitt des verjüngten distalen Bereichs angeordnet ist.

17. Spulenanordnung nach Anspruch 16, die ferner ein inneres Element aufweist, das innerhalb des zweiten Spulenelements angeordnet ist.

18. Spulenanordnung nach Anspruch 17, wobei das innere Element ein röhrenförmiges Element ist.

19. Spulenanordnung nach Anspruch 17 oder 18, wobei das innere Element aus einem Polymer besteht.

20. Spulenanordnung nach einem der Ansprüche 16 bis 19, die ferner ein äußeres Element aufweist, das um das erste Spulenelement angeordnet ist.

21. Spulenanordnung nach Anspruch 20, wobei das äußere Element aus einem Polymer besteht.

22. Spulenanordnung nach einem der Ansprüche 16 bis 21, wobei das erste Spulenelement einen ersten Spulenzwischenraum aufweist und das zweite Spulenelement einen zweiten Spulenzwischenraum aufweist, der fünf Mal größer als der erste Spulenzwischenraum ist.

23. Spulenanordnung nach einem der Ansprüche 16 bis 22, wobei der Kerndraht ferner einen proximalen Abschnitt aufweist, der einen Außendurchmesser aufweist, und das erste Spulenelement einen Außendurchmesser aufweist, der gleich dem Außendurchmesser des proximalen Abschnitts ist.

24. Verfahren zum Herstellen einer Spulenanordnung zur Verwendung in einer medizinischen Vorrichtung, wobei das Verfahren aufweist:
Bereitstellen eines ersten Spulenelements, das aus einem runden Draht gebildet ist, der in eine erste Richtung gewickelt ist und mehrere Spulenwindungen definiert, die einen offenen Zwischenraum aufweisen, wobei das erste Spulenelement ein Lumen definiert;
Bereitstellen eines zweiten Spulenelements, das aus einem Band gebildet ist, das in eine zweite, zur ersten Richtung unterschiedliche Richtung gewickelt ist und mehrere Spulenwindungen definiert, die einen offenen Zwischenraum aufweisen;
Anordnen eines Abschnitts des zweiten Spulenelements innerhalb des Lumens des ersten Spulenelements, so dass sich Spulenwindungen des ersten Spulenelements mit Spulenwindungen des zweiten Spulenelements überschneiden, um mehrere beabstandete Spulenüberschneidungsbereiche zu erzeugen; und
Befestigen des ersten Spulenelements am zweiten Spulenelement an mehreren der Überschneidungsbereiche.

25. Verfahren nach Anspruch 24, wobei das erste Spulenelement am zweiten Spulenelement an 25% oder mehr der Spulenüberschneidungsbereiche befestigt ist.

26. Verfahren nach Anspruch 24, wobei das erste Spulenelement am zweiten Spulenelement an einer Mehrzahl der Spulenüberschneidungsbereiche befestigt ist.

27. Verfahren nach Anspruch 24, wobei das erste Spulenelement am zweiten Spulenelement an 75% oder mehr der Spulenüberschneidungsbereiche befestigt ist.

28. Verfahren nach Anspruch 24, wobei das erste Spulenelement am zweiten Spulenelement an 90% oder mehr der Spulenüberschneidungsbereiche befestigt ist.

29. Verfahren nach Anspruch 24, wobei das erste Spulenelement am zweiten Spulenelement im Wesentlichen an allen Spulenüberschneidungsbereichen befestigt ist.

30. Verfahren nach Anspruch 24, das ferner aufweist:
Befestigen eines länglichen Schafts am ersten oder zweiten Spulenelement, wobei das erste Spulenelement eine äußere Spule umfasst und wobei das zweite Spulenelement eine innere Spule umfasst.

31. Verfahren nach Anspruch 30, wobei das Anordnen der äußeren Spule um die innere Spule das Anordnen der äußeren Spule um und in direktem Kontakt mit der inneren Spule aufweist.

32. Verfahren nach Anspruch 30 oder 31, wobei die innere Spule ein erstes Filament und ein zweites Filament aufweist.

33. Verfahren nach Anspruch 32, wobei das erste Filament und das zweite Filament gemeinsam gewickelt sind, so dass Windungen des ersten Filaments zwischen Windungen des zweiten Filaments angeordnet sind.

34. Verfahren nach Anspruch 32 oder 33, wobei sich das erste Filament und das zweite Filament einander radial gegenüberliegen.

35. Verfahren nach einem der Ansprüche 30 bis 34, wobei das Befestigen der inneren Spule an der äußeren Spule das Anschweißen der inneren Spule an der äußeren Spule aufweist.

36. Verfahren nach Anspruch 35, wobei das Anschweißen der inneren Spule an der äußeren Spule Laserschweißen der inneren Spule an die äußere Spule aufweist.

37. Verfahren nach Anspruch 35, wobei das Anschweißen der inneren Spule an der äußeren Spule HF-Schweißen der inneren Spule an die äußere Spule aufweist.

38. Verfahren nach einem der Ansprüche 30 bis 34, wobei das Befestigen der inneren Spule an der äußeren Spule Löten der inneren Spule an die äußere Spule aufweist.

39. Verfahren nach einem der Ansprüche 30 bis 34, wobei das Befestigen der inneren Spule an der äußeren Spule Kleben der inneren Spule an die äußere Spule mit einem Klebemittel aufweist.

40. Verfahren nach einem der Ansprüche 30 bis 39, wobei die innere Spule einen ersten Spulenzwischenraum aufweist und die äußere Spule einen zweiten Spulenzwischenraum aufweist, der kleiner als der erste Spulenzwischenraum ist.

41. Verfahren nach Anspruch 40, wobei der erste Spulenzwischenraum mindestens fünf Mal größer als der zweite Spulenzwischenraum ist.

42. Verfahren nach einem der Ansprüche 30 bis 41, wobei das Befestigen der inneren Spule an der äußeren Spule mit mehreren Befestigungspunkten das Befestigen der inneren Spule an der äußeren Spule mit 10 oder mehr Befestigungspunkten aufweist.

## Revendications

1. Unité d'enroulement destinée à une utilisation dans un dispositif médical, ladite unité d'enroulement comprenant :
un premier élément d'enroulement constitué d'un fil à section ronde enroulé dans une première direction et définissant une pluralité de spires d'enroulement à pas ouvert, ledit premier élément d'enroulement définissant une lumière ; et
un deuxième élément d'enroulement constitué d'un ruban enroulé dans une deuxième direction différente de la première direction et définissant une pluralité de spires d'enroulement à pas ouvert, une partie du deuxième élément d'enroulement étant disposée dans la lumière du premier élément d'enroulement, de sorte que les spires du premier élément d'enroulement se croisent avec les spires du deuxième élément d'enroulement pour former une pluralité de surfaces d'intersection d'enroulements espacées, et que le premier élément d'enroulement est fixé au deuxième élément d'enroulement sur une pluralité de surfaces d'intersection.

2. Unité d'enroulement selon la revendication 1, où le premier enroulement est fixé au deuxième enroulement sur au moins 25 % des surfaces d'intersection d'enroulements.

3. Unité d'enroulement selon la revendication 1, où le premier enroulement est fixé au deuxième enroulement sur une majorité des surfaces d'intersection d'enroulements.

4. Unité d'enroulement selon la revendication 1, où le premier enroulement est fixé au deuxième enroulement sur au moins 75 % des surfaces d'intersection d'enroulements.

5. Unité d'enroulement selon la revendication 1, où le premier enroulement est fixé au deuxième enroulement sur au moins 90 % des surfaces d'intersection d'enroulements.

6. Unité d'enroulement selon la revendication 1, où le premier enroulement est fixé au deuxième enroulement sur sensiblement toutes les surfaces d'intersection d'enroulements.

7. Unité d'enroulement selon la revendication 1, comprenant en outre une tige oblongue, le premier élément d'enroulement étant disposé autour d'au moins une partie de la tige oblongue.

8. Unité d'enroulement selon la revendication 7, où le deuxième élément d'enroulement comprend un premier filament et un deuxième filament.

9. Unité d'enroulement selon la revendication 8, où le premier filament et le deuxième filament sont enroulés conjointement, de sorte que les spires du premier filament sont intercalées entre les spires du deuxième filament.

10. Unité d'enroulement selon la revendication 8 ou la revendication 9, où le premier filament et le deuxième filament sont opposés radialement l'un à l'autre.

11. Unité d'enroulement selon l'une des revendications 7 à 10, comprenant en outre un troisième enroulement disposé dans la lumière du premier enroulement.

12. Unité d'enroulement selon la revendication 11, où le troisième enroulement est enroulé dans la deuxième direction.

13. Unité d'enroulement selon l'une des revendications 7 à 12, où le deuxième élément d'enroulement est formé sur un ruban plat.

14. Unité d'enroulement selon l'une des revendications 7 à 13, où le premier élément d'enroulement et le deuxième élément d'enroulement sont tous les deux à pas d'enroulement ouvert.

15. Unité d'enroulement selon l'une des revendications 7 à 14, où le premier élément d'enroulement présente un premier pas d'enroulement et le deuxième élément d'enroulement présente un deuxième pas d'enroulement supérieur au premier pas d'enroulement.

16. Unité d'enroulement selon la revendication 1, comprenant en outre un fil d'âme ayant une zone distale amincie, le premier élément d'enroulement étant disposé autour d'au moins une partie de la zone distale amincie.

17. Unité d'enroulement selon la revendication 16, comprenant en outre un élément intérieur disposé à l'intérieur du deuxième élément d'enroulement.

18. Unité d'enroulement selon la revendication 17, où l'élément intérieur est un élément tubulaire.

19. Unité d'enroulement selon la revendication 17 ou la revendication 18, où l'élément intérieur est polymérique.

20. Unité d'enroulement selon l'une des revendications 16 à 19, comprenant en outre un élément extérieur disposé autour du premier élément d'enroulement.

21. Unité d'enroulement selon la revendication 20, où l'élément extérieur est polymérique.

22. Unité d'enroulement selon l'une des revendications 16 à 21, où le premier élément d'enroulement présente un premier pas d'enroulement et le deuxième élément d'enroulement un deuxième pas d'enroulement cinq fois supérieur au premier pas d'enroulement.

23. Unité d'enroulement selon l'une des revendications 16 à 22, où le fil d'âme comporte en outre une partie proximale ayant un diamètre extérieur, et où le premier élément d'enroulement a un diamètre extérieur égal au diamètre extérieur de la partie proximale.

24. Procédé de fabrication d'une unité d'enroulement destinée à une utilisation dans un dispositif médical, ledit procédé comprenant :
la préparation d'un premier élément enroulé constitué d'un fil à section ronde enroulé dans une première direction et définissant une pluralité de spires d'enroulement à pas ouvert, ledit premier élément d'enroulement définissant une lumière ;
la préparation d'un deuxième élément enroulé constitué d'un ruban enroulé dans une deuxième direction différente de la première direction et définissant une pluralité de spires d'enroulement à pas ouvert ;
la disposition d'une partie du deuxième élément d'enroulement dans la lumière du premier élément d'enroulement, de sorte que les spires du premier élément d'enroulement se croisent avec les spires du deuxième élément d'enroulement pour former une pluralité de surfaces d'intersection d'enroulements espacées ; et
la fixation du premier élément d'enroulement au deuxième élément d'enroulement sur une pluralité des surfaces d'intersection.

25. Procédé selon la revendication 24, où le premier élément d'enroulement est fixé au deuxième élément d'enroulement sur au moins 25 % des surfaces d'intersection d'enroulements.

26. Procédé selon la revendication 24, où le premier élément d'enroulement est fixé au deuxième élément d'enroulement sur une majorité des surfaces d'intersection d'enroulements.

27. Procédé selon la revendication 24, où le premier élément d'enroulement est fixé au deuxième élément d'enroulement sur au moins 75 % des surfaces d'intersection d'enroulements.

28. Procédé selon la revendication 24, où le premier élément d'enroulement est fixé au deuxième élément d'enroulement sur au moins 90 % des surfaces d'intersection d'enroulements.

29. Procédé selon la revendication 24, où le premier élément d'enroulement est fixé au deuxième élément d'enroulement sur sensiblement toutes les surfaces d'intersection d'enroulements.

30. Procédé selon la revendication 24, comprenant en outre :
la fixation d'une tige oblongue au premier ou au deuxième élément d'enroulement, le premier élément d'enroulement présentant un enroulement extérieur et le deuxième élément d'enroulement présentant un enroulement intérieur.

31. Procédé selon la revendication 30, où la disposition de l'enroulement extérieur autour de l'enroulement intérieur comprend la disposition de l'enroulement extérieur autour, et au contact direct de l'enroulement intérieur.

32. Procédé selon la revendication 30 ou la revendication 31, où l'enroulement intérieur comprend un premier filament et un deuxième filament.

33. Procédé selon la revendication 32, où le premier filament et le deuxième filament sont enroulés conjointement, de sorte que les spires du premier filament sont intercalées entre les spires du deuxième filament.

34. Procédé selon la revendication 32 ou la revendication 33, où le premier filament et le deuxième filament sont opposés radialement l'un à l'autre.

35. Procédé selon l'une des revendications 30 à 34, où la fixation de l'enroulement intérieur à l'enroulement extérieur comprend le soudage de l'enroulement intérieur sur l'enroulement extérieur.

36. Procédé selon la revendication 35, où le soudage de l'enroulement intérieur sur l'enroulement extérieur comprend le soudage laser de l'enroulement intérieur sur l'enroulement extérieur.

37. Procédé selon la revendication 35, où le soudage de l'enroulement intérieur sur l'enroulement extérieur comprend le soudage RF de l'enroulement intérieur sur l'enroulement extérieur.

38. Procédé selon l'une des revendications 30 à 34, où la fixation de l'enroulement intérieur à l'enroulement extérieur comprend le soudage de l'enroulement intérieur sur l'enroulement extérieur.

39. Procédé selon l'une des revendications 30 à 34, où la fixation de l'enroulement intérieur à l'enroulement extérieur comprend la liaison de l'enroulement intérieur à l'enroulement extérieur au moyen d'un adhésif.

40. Procédé selon l'une des revendications 30 à 39, où l'enroulement intérieur présente un premier pas d'enroulement et l'enroulement extérieur un deuxième pas d'enroulement inférieur au premier pas d'enroulement.

41. Procédé selon la revendication 40, où le premier pas d'enroulement est au moins cinq fois supérieur au deuxième pas d'enroulement.

42. Procédé selon l'une des revendications 30 à 41, où la fixation de l'enroulement intérieur à l'enroulement extérieur par une pluralité de points de fixation comprend la fixation de l'enroulement intérieur à l'enroulement extérieur par au moins 10 points de fixation.
